# EUROPEAN PATENT APPLICATION

(11) **EP 4 349 996 A1**
(43) Date of publication of application: **10.04.2024**
(21) Application number: 22816037.0
(22) Date of filing: 30.05.2022
(51) Int. Cl.: C12P 21/06, C07K 1/12, C07K 9/00, C07K 14/465, C07K 16/02

(54) **METHOD FOR PRODUCING GLYCOPEPTIDE**

(30) Priority: 31.05.2021 JP 2021091853
(71) Applicant: KH Neochem Co., Ltd., Tokyo 103-0022 (JP)
(72) Inventor: NAKAMACHI, Yuto, Kawasaki-shi, Kanagawa 212-0032 (JP); TANAKA, Shinji, Kawasaki-shi, Kanagawa 212-0032 (JP); TOTANI, Kiichiro, Musashino-shi, Tokyo 180-8633 (JP); KURIBARA, Taiki, Musashino-shi, Tokyo 180-8633 (JP)
(74) Representative: MacLean, Martin Robert
(86) International application number: PCT/JP2022/021875
(87) International publication number: WO 2022/255289

(57) **Abstract**

The present invention relates to a method for producing a glycopeptide comprising steps of: denaturing the Fc region of an avian antibody; and degrading the denatured Fc region with a proteolytic enzyme; wherein the glycopeptide is a glycodipeptide or glycotripeptide.

## Description

### Technical Field

The present invention relates to method for producing a glycopeptide.

### Background Art

Glycans are biopolymers in which monosaccharides are linked in a branched form, and they have a wide variety of structures because each monosaccharide as a basic binding unit has a plurality of binding points.

In vivo glycans are present on the surfaces of proteins and cells, and are involved in protein and cell recognition and signal transduction.

In particular, antibodies, which are active ingredients of antibody drugs, usually have a glycan structure, and variations in the glycan structure lead to occurrence of adverse effects and variations in drug efficacy. Therefore, Making the glycan structure homogeneous has been expected to lead to improvement in the quality and function of the antibody drugs, such as suppression of adverse effects, improvement in drug efficacy and extension of half-life in blood.

Abnormalities in glycan structures are known to be associated with diseases such as a cancer, and utilization of glycans has been attempted to elucidate mechanisms of diseases. In addition to elucidation of mechanisms of diseases, application to diagnostic agents has also been attempted.

Homogeneous glycans have been being able to be obtained by organic synthesis. However, stereoselective and regioselective organic synthesis of glycans depending on a variety of the glycan structures requires multi-step reactions and is limited in the amount of the glycans to be synthesized.

Patent Literature 1 discloses, as derivatives capable of introducing a natural glycan into a specific substrate, a Fmoc (9-fluorenylmethyloxycarbonyl)-glycoasparagine obtained by subjecting a naturally occurring glycoasparagine to Fmoc addition and an active ester derivative derived from the Fmoc-glycoasparagine.

Non Patent Literature 1 discloses a powerful method for producing a glycoprotein having a single oligosaccharide using a Fmoc-Asn high-mannose glycan-type oligosaccharide. It also discloses that erythropoietin (positions 22 to 28) and interleukin 13 (positions 28 to 43) having a single oligosaccharide were actually prepared.

Non Patent Literature 2 discloses a method for preparing the asparagine-linked glycan of G1M9, which is a monoglycosylated high-mannose glycan-type glycan, from IgY in egg yolk.

Patent Literature 2 discloses a method for purifying a complex-type glycan having 11 to 5 monosaccharides from defatted egg yolk.

Patent Literatures 3 and 4 disclose a method for producing a 11-sugar sialyl oligosaccharide peptide.

### Citation List

### Patent Literature

Patent Literature 1: Japanese Patent Laid-Open No. 11-255807
Patent Literature 2: Japanese Patent No. 4237752
Patent Literature 3: Japanese Patent No. 5680576
Patent Literature 4: Japanese Patent No. 5566226

### Non Patent Literature

Non Patent Literature 1: Carbohydrate Research (2012) vol. 364, pp. 41-48
Non Patent Literature 2: Carbyhydrate Research (2015) vol. 411, pp. 37-41

### Summary of Invention

### Technical Problem

However, in the prior art described above, as for a high-mannose-type glycan, production of a glycoasparagine in which only asparagine is linked has been only achieved. As for a peptide linked to a glycan, the sequence of Asn-X-Thr/Ser wherein X represents any amino acid other than proline has been suggested, but no example of a high-mannose-type glycopeptide containing that sequence had been isolated have been found.

The problem to be solved by the present application is to provide a method for producing a novel glycopeptide.

### Solution to Problem

After diligent research, the present inventors have found a method for producing a novel glycopeptide by obtaining the glycopeptide from the Fc region of an avian antibody, and have completed the present invention.

That is, the present invention is as follows:
[1] A method for producing a glycopeptide, comprising steps of:
   denaturing the Fc region of an avian antibody; and
   degrading the denatured Fc region with a proteolytic enzyme;
   wherein the glycopeptide is a glycodipeptide or glycotripeptide.
[2] The method according to [1], wherein the glycan of the glycopeptide has 8 to 12 monosaccharides.
[3] The method according to [1] or [2], wherein in the step of denaturing the Fc region of an avian antibody, the Fc region is denatured with heat or an organic solvent.
[4] The method according to any of [1] to [3], wherein the avian antibody is IgY.
[5] A method for producing a glycan or glycoasparagine, further comprising a step of degrading the glycodipeptide or glycotripeptide obtained by the method according to any of [1] to [4] with a degrading enzyme.
[6] A method for separating and purifying a glycopeptide, comprising steps of:
   denaturing the Fc region of an avian antibody; and
   degrading the denatured Fc region with a proteolytic enzyme;
   wherein the glycopeptide is a glycodipeptide or glycotripeptide.
[7] A glycodipeptide or glycotripeptide derived from the Fc region of an avian antibody,
   wherein the glycodipeptide or glycotripeptide has a glycan of 8 to 12 monosaccharides.
[8] A glycotripeptide derived from the Fc region of an avian antibody,
   wherein the glycotripeptide is an N-glycotripeptide having a glycan of 8 to 12 monosaccharides in which the tripeptide has Asn-Gly-Thr/Ser and the glycan is linked to Asn, wherein one or more residues of Asn, Thr and Ser are optionally protected.
[9] A glycodipeptide derived from the Fc region of an avian antibody,
   wherein the glycodipeptide is an N-glycodipeptide having a glycan of 8 to 12 monosaccharides in which the dipeptide has Asn-Gly and the glycan is linked to Asn, wherein one or more residues of Asn and Gly are optionally protected.

### Advantageous Effects of Invention

The present invention can provide a method for producing a novel glycopeptide by obtaining the glycopeptide from the Fc region of an avian antibody. The present invention can also provide a novel glycodipeptide and glycotripeptide.

### Brief Description of Drawings

[Figure 1] Figure 1 shows mass peaks of glycotripeptide derivatives having 8 to 12 monosaccharides in the mass spectrometry.
[Figure 2] Figure 2 shows mass peaks of glycodipeptide derivatives having 8 to 12 monosaccharides in the mass spectrometry.
[Figure 3] Figure 3 shows the results of mass spectrometry of glycotripeptide derivatives each different in the number of monosaccharides obtained by fractionation.
[Figure 4] Figure 4 shows ¹H-NMR data of a glycotripeptide derivative having 12 monosaccharides (Glc1Man9GlcNAc2-Asn(Fmoc)-Gly-Thr).
[Figure 5] Figure 5 shows the results of mass spectrometry of a glycodipeptide derivative having 12 monosaccharides (Glc1Man9GlcNAc2-Asn(Fmoc)-Gly).
[Figure 6] Figure 6 shows ¹H-NMR data of a glycodipeptide derivative having 12 monosaccharides (Glc1Man9GlcNAc2-Asn(Fmoc)-Gly).
[Figure 7] Figure 7 shows the mass peak of a glycoasparagine derivative having 12 monosaccharides (Glc1Man9GlcNAc2-Asn-Fmoc) in the mass spectrometry.
[Figure 8] Figure 8 shows the mass peak of a glycan having 12 monosaccharides (Glc1Man9GlcNAc2) in the mass spectrometry.
[Figure 9] Figure 9 shows the mass peak of a glycan having 11 monosaccharides in the mass spectrometry.
[Figure 10] Figure 10 shows the result of confirmation by mass spectrometry that cleavage of the glycan of the isolated and purified glycoasparagine derivative having 11 monosaccharides has not progressed.

### Description of Embodiments

The embodiments of the present invention will be described in detail below. The present invention is not limited to the following embodiments, but can be variously modified and carried out within the spirit thereof.

The method for producing a glycopeptide according to the present invention comprises steps of:
denaturing the Fc region of an avian antibody; and
degrading the denatured Fc region with a proteolytic enzyme.
The glycopeptide obtained by the method for producing a glycopeptide of the present invention is a glycodipeptide or glycotripeptide.

### (Glycodipeptide or glycotripeptide)

The glycodipeptide or glycotripeptide produced by the present invention is a glycopeptide derived from the Fc region of an avian antibody, and they therefore have the structures present in the Fc region of an avian antibody.

That is, according to the present invention, the glycan present in the Fc region of an avian antibody can be obtained as a glycodipeptide or glycotripeptide.

The glycodipeptide is preferably a glycodipeptide derived from the Fc region of an avian antibody, and the glycotripeptide is preferably a glycotripeptide derived from the Fc region of an avian antibody.

The peptide moiety of the glycodipeptide or glycotripeptide is a glycodipeptide or glycotripeptide containing amino acids to which the glycan in the Fc region of an avian antibody is linked, respectively.

Therefore, since the glycodipeptide and glycotripeptide produced by the present invention are obtained by cutting out the glycan present in the Fc region of the avian antibody as a glycodipeptide and glycotripeptide, respectively, the structures are derived from a glycan present in the Fc region of an avian antibody and a peptide sequence composed of an amino acid to which the glycan is linked and an amino acid to which the former amino acid is linked.

The structure of the glycan moiety of the glycodipeptide or glycotripeptide is not limited as long as it is the structure of the glycan moiety present in the Fc region of an avian antibody, but the glycan is preferably a glycan of 8 to 12 monosaccharides.

The glycodipeptide is preferably a glycodipeptide, having a glycan of 8 to 12 monosaccharides, derived from the Fc region of an avian antibody, and the glycotripeptide is preferably a glycotripeptide, having a glycan of 8 to 12 monosaccharides, derived from the Fc region of an avian antibody.

The glycan of 8 to 12 monosaccharides may be a glycan known as a high-mannose-type glycan.

Examples of the high-mannose-type glycan include glycans shown in the following schematic diagram.

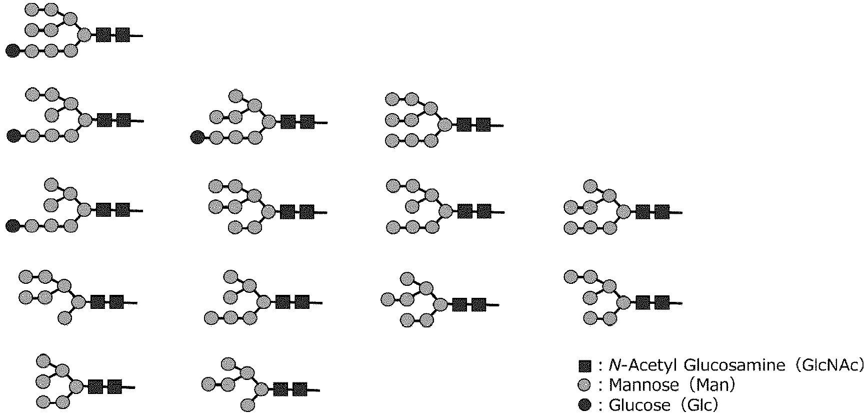

Fourteen types of structures are shown as the structures of glycans having 8 to 12 monosaccharides.

The bonding hand on the right side of the two squares linked to each other is a bonding hand with a peptide. Each of the two squares means N-acetylglucosamine, and the N-acetylglucosamine present on the right side forms the so-called reducing terminal of the glycan.

As an example of the glycan, the glycan having 12 monosaccharides will be described below.

For the glycan having 12 monosaccharides in the above schematic diagram, three arms are referred to as C-arm, B-arm and A-arm from the top, and the non-reducing terminal of the A-arm is glucose.

All monosaccharides other than the glucose at the non-reducing terminal of the A-arm and the two N-acetylglucosamines at the reducing terminal are mannose.

Each bond between the monosaccharides is not limited as long as it is a bond present in the structure derived from the Fc region of an avian antibody, and it may be an α-bond or a β-bond. The position of the hydroxyl group to which the glycan binds is also not limited as long as it is a position present in the structure derived from the Fc region of the avian antibody, and it may be 1→2 bond, 1→3 bond, 1→4 bond or 1→6 bond.

Each of N-acetylglucosamine, glucose and mannose is typically a D-sugar.

The glycan of 8 to 12 monosaccharides is preferably a glycan having the structure specifically shown below. For convenience, the amino acid to which N-acetylglucosamine at the reducing terminal is linked is described as Asn (asparagine). The compound described below as a specific example is a glycoasparagine having 8 to 12 monosaccharides.

The glycopeptide having N-acetylglucosamine linked to Asn is also referred to as an N-glycopeptide, which may be also referred to as an N-glycotripeptide, an N-glycodipeptide or an N-glycoasparagine, depending on the number of amino acids to which the glycan is linked.

When the glycan has 12 monosaccharides, the glycan may have the following structure.

In the following structure, for example, α1→2 means that the mannose on the non-reducing terminal side is α-bound at position 1 thereof to the hydroxyl group at position 2 of the mannose on the reducing terminal side. β1→N means that the N-acetylglucosamine at the reducing terminal is β-bound at position 1 thereof to N of Asn in the peptide of the glycopeptide. The same applies to glycans having 8 to 11 monosaccharides shown below.

### Glc1Man9GlcNAc2

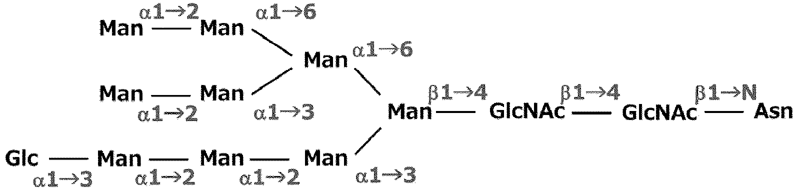

When the glycan has 11 monosaccharides, the glycan may have the following structure.

### Glc1Man8GlcNAc2

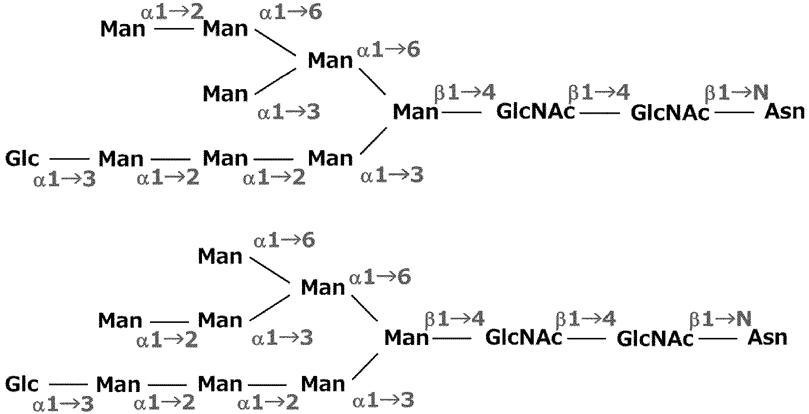

### Man9GlcNAc2

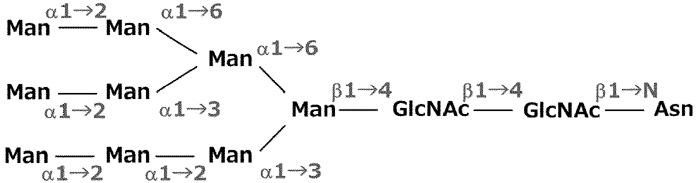

When the glycan has 10 monosaccharides, the glycan may have the following structure.

### Glc1Man7GlcNAc2

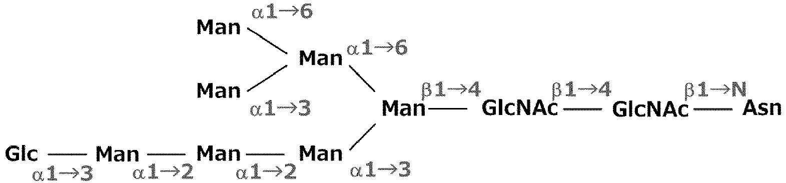

### Man8GlcNAc2

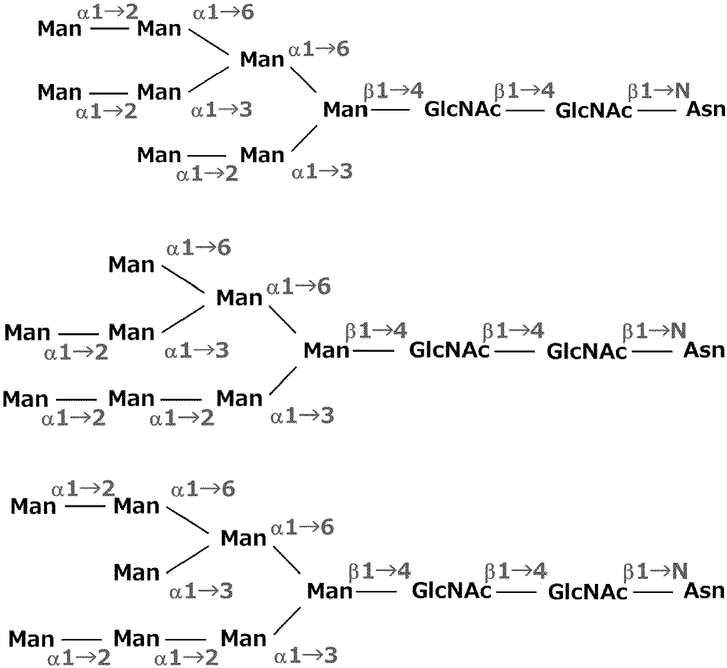

When the glycan has 9 monosaccharides, the glycan may have the following structure.

### Man7GlcNac2

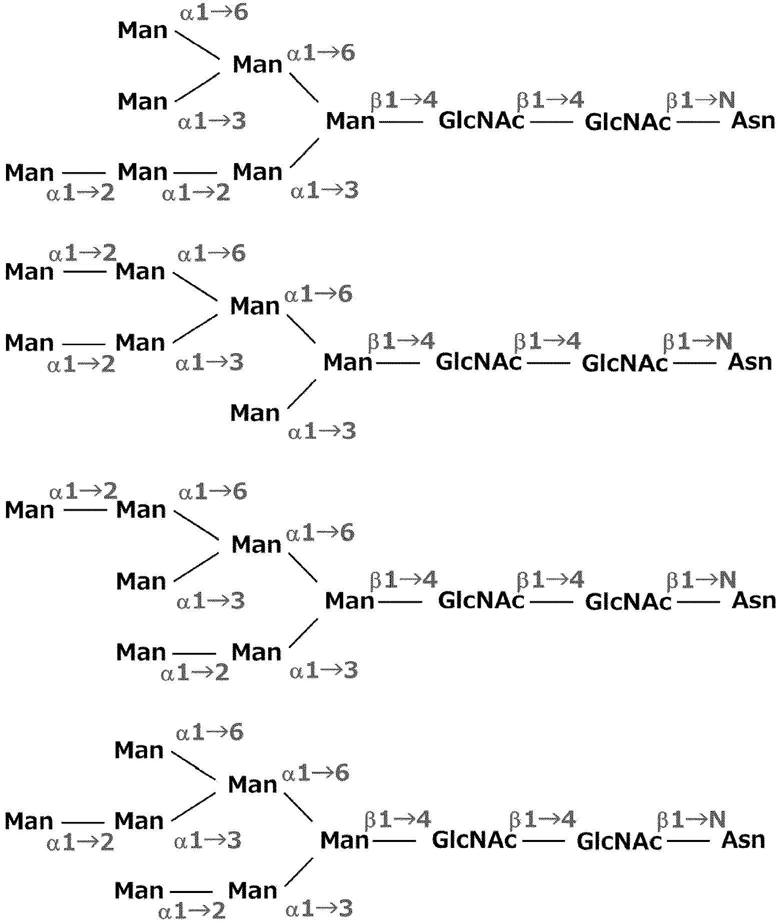

When the glycan has 8 monosaccharides, the glycan may have the following structure.

### Man6GlcNAc2

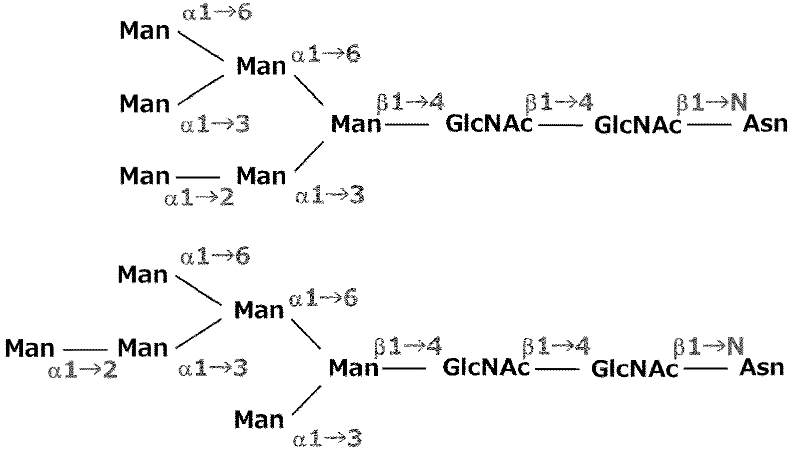

The structure of the peptide moiety of the glycodipeptide or glycotripeptide is not limited as long as it is the structure of the peptide moiety present in the Fc region of an avian antibody, but the peptide moiety is preferably a dipeptide or tripeptide containing Asn.

Asn in the dipeptide may be either an N-terminal amino acid or a C-terminal amino acid, but is preferably an N-terminal amino acid.

The other amino acid of the dipeptide is not limited as long as it is an amino acid derived from the structure of the peptide moiety present in the Fc region of an avian antibody, but it is preferably Gly (glycine).

Each of Asn and Gly may be either an L-amino acid or a D-amino acid, but are preferably an L-amino acid, and the dipeptide preferably has a dipeptide-derived structure of H₂N-Asn-Gly-COOH. Here, H₂N- is an amino group present in Asn, and -COOH is a carboxyl group present in Gly.

Asn in the tripeptide may be an N-terminal amino acid, a C-terminal amino acid, or an amino acid located in the center, but it is preferably an N-terminal amino acid.

The tripeptide having Asn at the N-terminal preferably has the dipeptide-derived structure of H₂N-Asn-Gly-COOH described above. In this case, the C-terminal amino acid is not limited, but it is preferably Thr (threonine) or Ser (serine) as the structure of the peptide moiety present in the Fc region of an avian antibody.

Each of Asn and Gly and Thr or Ser may be an L-amino acid or a D-amino acid, but it is preferably an L-amino acid. The tripeptide preferably has a tripeptide-derived structure of H₂N-Asn-Gly-Thr/Ser-COOH. Here, H₂N- is an amino group present in Asn, and -COOH is a carboxyl group present in Thr or Ser.

The glycotripeptide is preferably a glycotripeptide derived from the Fc region of an avian antibody, and preferably an N-glycotripeptide having a glycan of 8 to 12 monosaccharides in which the tripeptide has Asn-Gly-Thr/Ser and the glycan is linked to Asn.

In the glycotripeptide, one or more residues of Asn, Thr and Ser are optionally protected.

The glycodipeptide is preferably a glycodipeptide derived from the Fc region of an avian antibody, and preferably an N-glycodipeptide having a glycan of 8 to 12 monosaccharides in which the dipeptide has Asn-Gly and the glycan is linked to Asn.

In the glycodipeptide, one or more residues of Asn and Gly are optionally protected.

When one or more residues of Asn, Thr and Ser are protected or when one or more residues of Asn and Gly are protected, the term "protected" means that each amino acid is protected by any amino acid protecting group as conventionally known. When one or more residues of Asn, Gly, Thr and Ser are protected, the glycopeptide is also referred to as a glycoasparagine derivative, a glycodipeptide derivative or a glycotripeptide derivative, which may be included in the glycoasparagine, glycodipeptide or glycotripeptide of the present invention, respectively.

Asn, which is L-Asn, has the following structure and the glycan is linked thereto. The glycoasparagine is bound to the CONH₂ group in the amino acid side chain, and for example, when Asn is an N-terminal amino acid, the COOH group of Asn forms an amide bond with the NH₂ group of Gly to form a dipeptide.

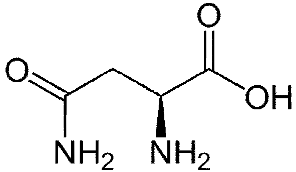

When the Asn is an amino acid present at the N-terminal, the remaining amino group (NH₂ group) may be protected because remaining NH₂ group may be present in the free form.

Gly has the following structure. When Gly is an amino acid present at the C-terminal, the NH₂ group of Gly forms an amide bond with the COOH group of Asn.

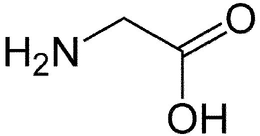

Therefore, the carboxyl group (COOH group) may be protected because remaining COOH group may be present in the free form.

The Ser or Thr, which is an L-Ser or L-Thr, respectively, has the following structure. When Ser or Thr is an amino acid present at the C-terminal, the NH₂ of Ser or Thr forms an amide bond with the COOH group of Gly.

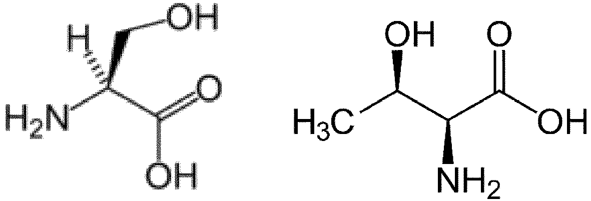

When the Ser or Thr is an amino acid present at the C-terminal, because the remaining carboxyl group (COOH group) may be present in the free form and a hydroxyl group (OH group) is present, the COOH group and the OH group may therefore be protected.

The protecting groups to be utilized for each of the amino group, carboxyl group and hydroxyl group can be any known protecting groups. For example, the protecting groups described in Greene's Protective Groups in Organic Synthesis may be selected.

Examples of the protecting group for an amino group include an Fmoc group, a t-butoxycarbonyl (Boc) group, a benzyloxycarbonyl (Cbz or Z) group, a p-methoxybenzyloxycarbonyl (Z(OMe) or pMZ) group and a 2-(p-biphenyl)isopropyloxycarbonyl (Bpoc) group.

Examples of the protecting for a carboxyl group include a methyl ester group, an ethyl ester group, a benzyl ester group and a t-butyl ester group. The methyl ester group, the ethyl ester group, the benzyl ester group and the t-butyl ester group are protecting groups formed by forming an ester between a carboxyl group and methyl alcohol, ethyl alcohol, benzyl alcohol and t-butyl alcohol, respectively.

Examples of the protecting group for a hydroxyl group include an acetyl (Ac) group, a benzoyl (Bz) group, a methoxymethyl (MOM) group, a benzyl (Bn) group and a t-butyl (t-Bu) group. The protecting group for a hydroxyl group may be a group known as a silyl-based protecting group such as a trimethylsilyl (TMS) group, a triethylsilyl (TES) group, a t-butyldimethylsilyl (TBS) group, a triisopropylsilyl (TIPS) group and a t-butyldiphenylsilyl (TBDPS) group.

The amino group or carboxyl group of the glycodipeptide or glycotripeptide may be present in the free form (NH₂ or COOH), may be present as NH₃⁺ or COO⁻, or may be a combination thereof.

The amino group or carboxyl group of the glycodipeptide or glycotripeptide may be also in the form of a salt. When the amino group or carboxyl group of the glycodipeptide or glycotripeptide is in the form of a salt, the salt is not limited. For example, the salt may be an inorganic acid salt such as hydrochloride, sulfate or phosphate, or an organic acid salt such as acetate, propionate, tartrate, fumarate, maleate, malate, citrate, methanesulfonate, p-toluenesulfonate or trifluoroacetate. For example, the salt may be also an alkali metal salt or an alkali earth metal salt such as a sodium salt, a potassium salt, a calcium salt or a magnesium salt.

The method for producing a glycopeptide according to the present invention comprises a step of denaturing the Fc region of an avian antibody.

The Fc region of an avian antibody is a region, constituting a heavy chain, which has an effector function that induces a reaction after an antibody has bound to an antigen.

In the present invention, a glycopeptide derived from the Fc region of an avian antibody is produced, and the Fc region can be therefore utilized without limitation as long as the glycopeptide can be cut off from the Fc region.

That is, the Fc region to be denatured in the present invention may be all or part of the entire Fc region. The Fc region to be used may be a Fc fragment if the glycopeptide can be obtained by denaturing it, or the glycopeptide may be produced directly from an avian antibody instead of using the Fc region as a fragment.

The step of denaturing the Fc region of an avian antibody may be a step of denaturing the Fc fragment of an avian antibody, or may be a step of denaturing the Fc region that has not been fragmented from the avian antibody, that is, the avian antibody itself.

The Fc fragment is not limited, and an avian antibody may be obtained by any conventionally known method and its Fc fragment may be used.

The avian antibody is not limited as long as it is derived from an avian, and the entire amino acid sequence of the antibody is derived from the avian.

Examples of the avian include a chicken, a goose and a duck, and it is preferably a chicken.

Examples of the isoform of the avian antibody include, but are not limited to, IgY, IgA and IgM, and it is preferably IgY.

The avian antibody can be obtained from an avian egg or blood by any conventionally known method. Among them, a lot of IgY can be obtained from a chicken egg, and an antibody derived from the chicken egg may be therefore utilized.

The Fc fragment of the avian antibody can be obtained, for example, by subjecting IgY as an avian antibody to papain digestion.

### (Denaturation of Fc region)

The method for denaturing the Fc region of an avian antibody refers to a method for changing the conformation of the Fc region. As a result of changing the conformation of the Fc region to change its reactivity with proteolytic enzyme, glycopeptides different in the number of amino acids can be obtained when the Fc region is reacted with proteolytic enzyme.

The method for denaturing the Fc region of an avian antibody is not limited as long as the conformation of the Fc region can be changed. Examples thereof include a process for denaturing the Fc region of an avian antibody with heat or an organic solvent.

When the Fc region is denatured by heat treatment, the heat treatment is preferably performed under the following reaction conditions from the viewpoint of reactivity with a proteolytic enzyme.

The reaction temperature is not limited, but it is preferably 70°C to 100°C, more preferably 75°C to 95°C, and even more preferably 80°C to 90°C.

The reaction time is not limited, but it is preferably 1 to 20 minutes, and more preferably 5 to 10 minutes.

A solvent for a solution containing the Fc region of the avian antibody in the heat treatment, for example, is preferably water, and it may be a buffer solution or an aqueous solution.

When the Fc region is denatured by treatment with an organic solvent, examples of the organic solvent to be used include, but are not limited to, acetone, methanol, ethanol, 2-propanol, chloroform and ethyl acetate.

The organic solvent to be used may be one solvent or a mixed solvent of two or more.

When using a water-soluble organic solvent, the Fc region of the avian antibody may be denatured with an aqueous solution containing the organic solvent.

The reaction time is not limited, but it is preferably 1 to 24 hours, and more preferably 3 to 12 hours.

The reaction temperature is not limited, and may be, for example, room temperature.

In treatment with the organic solvent, the Fc region of the avian antibody may be directly immersed in or contacted with an organic solvent, but the organic solvent may be added to a solution containing the Fc region of an avian antibody, or the solution containing the Fc region of an avian antibody may be added to the organic solvent.

### (Degradation of denatured Fc region)

The proteolytic enzyme to be used in the method for degrading the Fc region of the denatured avian antibody can be actinase E, pronase, orientase or the like, but it is preferably actinase E from the viewpoint of substrate specificity.

In the method for degrading the Fc region of the denatured avian antibody with a proteolytic enzyme, the reaction temperature is not limited but is 25°C to 50°C, and more preferably 35°C to 45°C.

The reaction time is not limited, but it is preferably 12 hours to 168 hours, more preferably 24 hours to 96 hours.

The denatured Fc region of an avian antibody can be degraded with a proteolytic enzyme to obtain a glycodipeptide or glycotripeptide.

The method for purifying the glycodipeptide or glycotripeptide after degradation with the proteolytic enzyme is not limited, and it is any conventionally known method.

After inactivating the proteolytic enzyme in the proteolytic enzyme-treated liquid, the liquid may be subjected to crude purification by column chromatography or the like to obtain a glycodipeptide or glycotripeptide.

The obtained glycodipeptide or glycotripeptide may be further purified after introducing a protecting group thereinto.

The reaction for introducing the protecting group into the glycodipeptide or glycotripeptide is not limited, and can be performed by any conventionally known method.

The purification of the glycodipeptide or glycotripeptide having the protecting group introduced thereinto is also not limited, and can be performed by any conventionally known method.

The glycodipeptide or glycotripeptide obtained by the method for producing a glycopeptide of the present invention can be further degraded with a degrading enzyme, preferably a proteolytic enzyme, to obtain a glycoasparagine.

Examples of the proteolytic enzyme include, but are not limited to, actinase E, carboxypeptidase and aminopeptidase, and it is preferably actinase E and carboxypeptidase.

The glycodipeptide or glycotripeptide obtained by the method for producing a glycopeptide of the present invention can be further degraded with a degrading enzyme to obtain a glycan having no amino acid bound to the reducing terminal.

The N-glycanase to be used is preferably PNGase F with no limitation.

The method for producing a glycopeptide of the present invention may be a method for separating and purifying a glycopeptide.

That is, as one aspect of the present invention, it may be a method for separating and purifying a glycopeptide, comprising steps of:
denaturing the Fc region of an avian antibody; and
degrading the denatured Fc region with a proteolytic enzyme;
wherein the glycopeptide is a glycodipeptide or glycotripeptide.
In the method for separating and purifying a glycopeptide, the particulars described for the method for producing a glycopeptide of the present invention can be applied as they are.

The glycopeptide obtained by the method of the present invention can be used as a research reagent, an industrial raw material and a pharmaceutical additive. For example, in folding diseases such as diabetes and Alzheimer's disease, it is known that the production ratio of the high-mannose-type glycan having 8 to 12 monosaccharides changes in vivo. The glycopeptide of the present invention can be therefore used as a glycopeptide-type probe for analyzing symptoms of such diseases. The glycopeptide is also expected to be applied as an antigen for an anti-glycan antibody and for adjusting the functions of a protein drug by introducing a glycan thereto.

The glycan is known to change in the functions and the recognition by an enzyme, depending on the number of amino acids bound thereto, and in particular, an arbitrary peptide containing a consensus sequence have been shown to have superior properties.

The consensus sequence is a sequence consisting of three amino acids necessary for the in vivo biosynthesis of an N-glycan on a protein, that is, Asn-X-Thr/Ser wherein X represents any amino acid other than proline.

In the present invention, it has been found that the activity of PNGase involved in the in vivo metabolism of glycans, differs in a glycoasparagine, a glycotripeptide and a glycodipeptide from each other. This reaction can also provide a glycan having no amino acid bound, for which efficient production has not yet been achieved. Therefore, the glycotripeptide and glycodipeptide obtained by the present invention have properties different from those of glycoasparagine, and provide effects that cannot be provided by the glycoasparagine.

### Examples

The present embodiment will be described below in more detail with reference to Examples and Comparative Examples, but the present embodiment is not limited only to these examples. The measurement method to be used is as follows.

### [HPLC analysis]

LC-2000 series PLUS available from JASCO Corporation
Column: INERTSIL Amide column 5 µm, 10 × 250 mm
Flow rate: 5 ml/min
UV: 273 nm
Gradient: CH₃CN/NH₄HCO₂ (100 mM, pH 4.5) = 65/35 → 50/50 (60 min)
[Mass spectrometry]
AXIMA-CFRplus available from SHIMADZU CORPORATION
Ionization: MALDI
Mode: Positive
[¹H-NMR]
JNE-ECA500 type FTNMR spectrometer (500 MHz) available from JEOL Ltd.
Solvent: D₂O

### <Example 1>

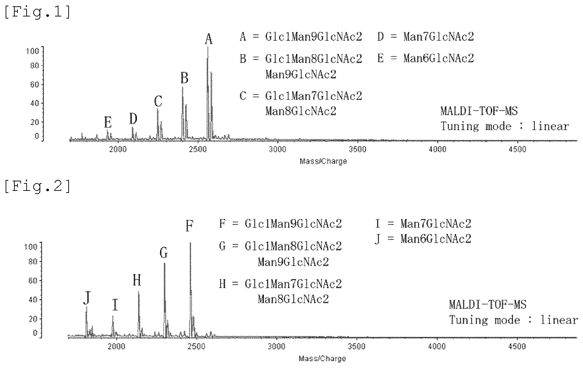

(a) Ethanol was added to about 180 mg of the fractions containing the Fc region of IgY in five times the amount of the fraction, and allowed to stand overnight. The precipitate was collected by centrifugation, dissolved in Tris-hydrochloric acid/calcium chloride buffer solution (pH=8.0, 90 mL), and sodium azide (0.38 mg) was added thereto. Further, actinase E (available from KAKEN PHARMACEUTICAL CO., LTD., 36 mg) was added thereto and allowed to react at 37°C for 48 hours.
(b) After completion of the reaction, the supernatant was collected by centrifugation. The supernatant was subjected to purification with a column packed with cotton under gradient conditions (CH₃CN/H₂O=90/10 → 0/100) to collect fractions containing glycopeptides.
(c) The collected fractions were concentrated, the resulting residue (about 11.6 mg) was dissolved in water/acetone solvent (water/acetone = 3/2, 1 mL) and sodium bicarbonate (3.9 mg) was added thereto. N-(9-Fluorenylmethoxycarbonyloxy)succinimide (Fmoc-OSu, 7.8 mg) was added thereto, allowed to react at room temperature for 6 hours, and concentrated with an evaporator. The concentrate was subjected to purification with a reversed-phase column (Sep-Pak C-18, available from Waters Corporation) under gradient conditions (H₂O/CH₃OH = 90/10 -> 60/40) to obtain the target high-mannose-type glycotripeptide derivatives (7.7 mg) having 8 to 12 monosaccharides. Mass peaks (Figure 1) of the target high-mannose-type glycotripeptide derivatives having 8 to 12 monosaccharides was confirmed by mass spectrometry.

### <Example 2>

(a) About 180 mg of the fractions containing the Fc region of IgY was incubated at 85°C for 10 minutes and adjusted to be Tris-hydrochloric acid/calcium chloride buffer solution (pH=8.0, 90 mL), and sodium azide (0.38 mg) was added thereto. Further, actinase E (available from KAKEN PHARMACEUTICAL CO., LTD., 36 mg) was added thereto and allowed to react at 37°C for 48 hours.
(b) After completion of the reaction, the supernatant was collected by centrifugation. The supernatant was subjected to purification with a column packed with cotton under gradient conditions (CH₃CN/H₂O=90/10 → 0/100) to collect fractions containing glycopeptides.
(c) The collected fractions were concentrated, the resulting residue (about 11.4 mg) was dissolved in water/acetone solvent (water/acetone = 3/2, 1 mL) and sodium bicarbonate (3.8 mg) was added thereto. N-(9-Fluorenylmethoxycarbonyloxy)succinimide (Fmoc-OSu, 7.5 mg) was added thereto, allowed to react at room temperature for 6 hours, and concentrated with an evaporator. The concentrate was subjected to purification with a reversed-phase column (Sep-Pak C-18, available from Waters Corporation) under gradient conditions (H₂O/CH₃OH = 90/10 -> 60/40) to obtain the target high-mannose-type glycotripeptide derivatives (7.5 mg) having 8 to 12 monosaccharides. Mass peaks (Figure 2) of the target high-mannose-type glycotripeptide derivatives having 8 to 12 monosaccharides was confirmed by mass spectrometry.

### <Example 3>

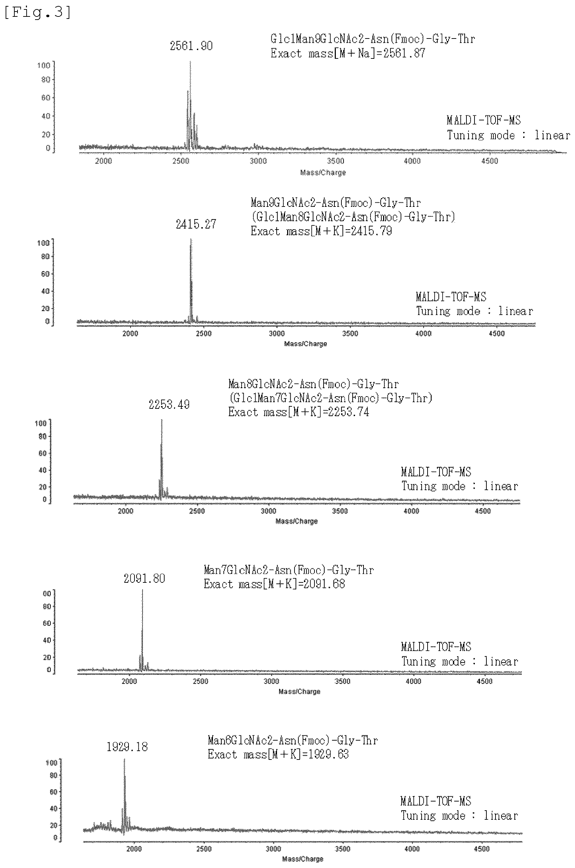

(a) The high-mannose-type glycotripeptide derivatives obtained in Example 1 were subjected to purification with a HPLC preparative column to obtain derivatives having 12, 11, 10, 9, or 8 monosaccharides. The results of mass spectrometry of glycotripeptide derivatives each different in the number of monosaccharides obtained by fractionation are shown (Figure 3).

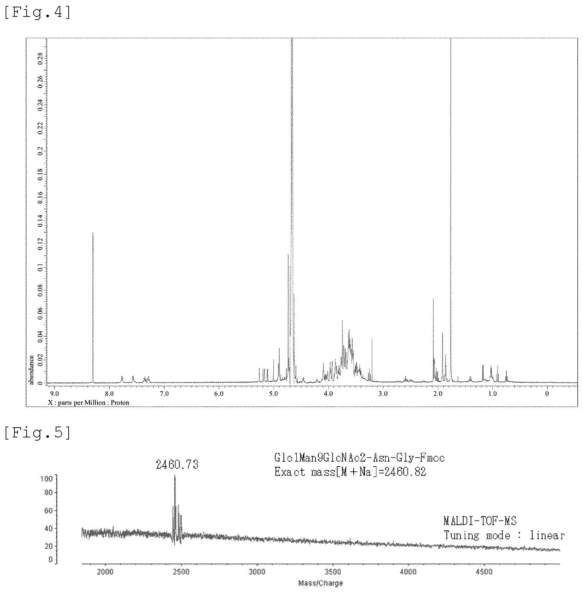

The main peak appearing after about 47 minutes was obtained by fractionation and was subjected to desalting treatment by gel filtration chromatography. It was concentrated under reduced pressure followed by vacuum drying to obtain about 2.6 mg of a glycotripeptide derivative having 12 monosaccharides (Glc1Man9GlcNAc2-Asn(Fmoc)-Gly-Thr). ¹H-NMR data of the obtained compound (Figure 4) are shown.

### <Example 4>

(a) The high-mannose-type glycodipeptide derivatives obtained in Example 2 were subjected to purification with a HPLC preparative column to obtain derivatives having 12, 11, 10, 9, or 8 monosaccharides.

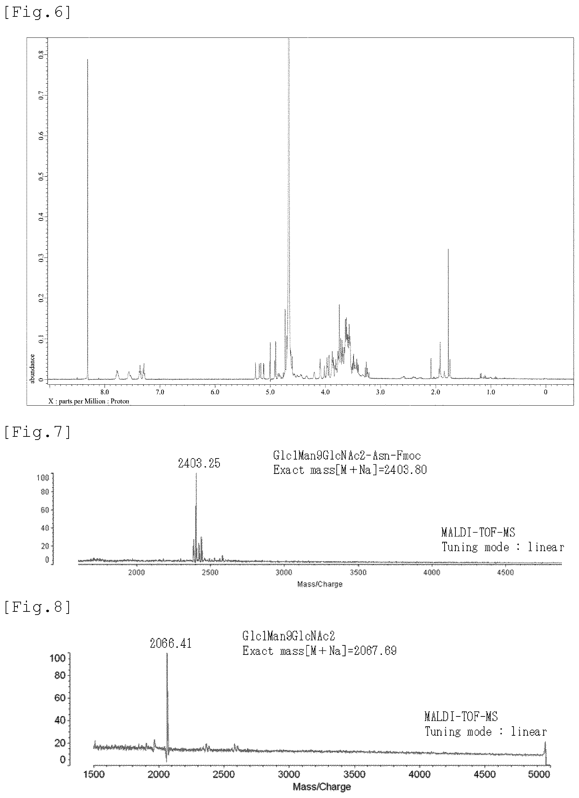

The main peak appearing after about 47 minutes was obtained by fractionation and was subjected to desalting treatment by gel filtration chromatography. It was concentrated under reduced pressure followed by vacuum drying to obtain about 2.4 mg of a glycodipeptide derivative having 12 monosaccharides (Glc1Man9GlcNAc2-Asn(Fmoc)-Gly. The results of mass spectrometry of the obtained compound (Figure 5) and ¹H-NMR data of the obtained compound (Figure 6) are shown.

### <Example 5>

(a) A 1.0 mg portion of the glycodipeptide derivative having 12 monosaccharides (Glc1Man9GlcNAc2-Asn(Fmoc)-Gly) was dissolved in Tris-hydrochloric acid/calcium chloride buffer solution (pH=8.0, 900 µL), and a 10% aqueous sodium azide solution was added thereto to adjust the molar concentration to 0.05%. Further, actinase E (available from KAKEN PHARMACEUTICAL CO., LTD., 0.2 mg) was added thereto and allowed to react at 37°C for 48 hours. The mass peak of the glycoasparagine derivative (Glc1Man9GlcNAc2-Asn-Fmoc) in which the amino acid had been cleaved was confirmed by mass spectrometry (Figure 7).

### <Example 6>

(a) A 1 µg portion of the isolated and purified glycotripeptide derivative having 12 monosaccharides (Glc1Man9GlcNAc2-Asn(Fmoc)-Gly-Thr) was dissolved in a reaction liquid (pH=7.5, 50 mM phosphate buffer, 1% NP-40), and PNGase F (available from New England BioLabs Inc., 4 µg) was added thereto and allowed to react at 37°C for 2 hours. The mass peak of the glycan having no peptide and 12 monosaccharides (Glc1Man9GlcNAc2) was confirmed by mass spectrometry (Figure 8).

### <Example 7>

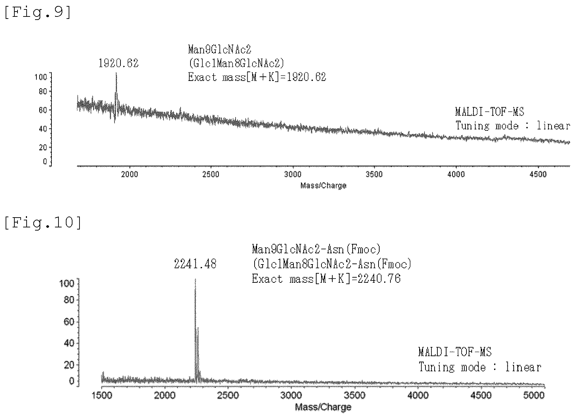

(a) A 1 µg portion of the isolated and purified glycodipeptide derivative having 11 monosaccharides was dissolved in a reaction liquid (pH=7.5, 50 mM phosphate buffer, 1% NP-40), and PNGase F (available from New England BioLabs Inc., 4 µg) was added thereto and allowed to react at 37°C for 2 hours. The mass peak of the glycan having no peptide and 12 monosaccharides was confirmed by mass spectrometry (Figure 9).

### <Example 8>

(a) A 1 µg portion of the isolated and purified glycoasparagine derivative having 11 monosaccharides was dissolved in a reaction liquid (pH=7.5, 50 mM phosphate buffer, 1% NP-40), and PNGase F (available from New England BioLabs Inc., 4 µg) was added thereto and allowed to react at 37°C for 2 hours. It was confirmed by mass spectrometry that the cleavage of the glycan had not progressed (Figure 10).

## Claims

1. A method for producing a glycopeptide, comprising steps of:
denaturing the Fc region of an avian antibody; and
degrading the denatured Fc region with a proteolytic enzyme;
wherein the glycopeptide is a glycodipeptide or glycotripeptide.

2. The method according to claim 1, wherein the glycan of the glycopeptide has 8 to 12 monosaccharides.

3. The method according to claim 1, wherein in the step of denaturing the Fc region of an avian antibody, the Fc region is denatured with heat or an organic solvent.

4. The method according to claim 1, wherein the avian antibody is IgY.

5. A method for producing a glycan or glycoasparagine, further comprising a step of degrading the glycodipeptide or glycotripeptide obtained by the method according to any one of claims 1 to 4 with a degrading enzyme.

6. A method for separating and purifying a glycopeptide, comprising steps of:
denaturing the Fc region of an avian antibody; and
degrading the denatured Fc region with a proteolytic enzyme;
wherein the glycopeptide is a glycodipeptide or glycotripeptide.

7. A glycodipeptide or glycotripeptide derived from the Fc region of an avian antibody,
wherein the glycodipeptide or glycotripeptide has a glycan of 8 to 12 monosaccharides.

8. A glycotripeptide derived from the Fc region of an avian antibody,
wherein the glycotripeptide is an N-glycotripeptide having a glycan of 8 to 12 monosaccharides in which the tripeptide has Asn-Gly-Thr/Ser and the glycan is linked to Asn, wherein one or more residues of Asn, Thr and Ser are optionally protected.

9. A glycodipeptide derived from the Fc region of an avian antibody,
wherein the glycodipeptide is an N-glycodipeptide having a glycan of 8 to 12 monosaccharides in which the dipeptide has Asn-Gly and the glycan is linked to Asn, wherein one or more residues of Asn and Gly are optionally protected.
